# EUROPÄISCHE PATENTSCHRIFT

(11) **EP 1 476 446 B1**
(45) Veröffentlichungstag und Bekanntmachung des Hinweises auf die Patenterteilung: **01.08.2007**
(21) Anmeldenummer: 03700112.0
(22) Anmeldetag: 17.01.2003
(51) Int. Cl.: C07D 487/04

(54) **VERFAHREN ZUR HERSTELLUNG VON N-ETHYL-N-(3-(3-CYANOPYRAZOLO-(1,5-A)-PYRIMIDIN-7-YL)PHENYL)-ACETAMID**
METHOD FOR PRODUCING N-ETHYL-N-( 3-(3-CYANOPYRAZOLO-(1,5A)-PYRIMIDINE-7-YL)PHENYL)-ACETAMIDE
PROCEDE DE FABRICATION DE N-ETHYL-N-(3-(3-CYANOPYRAZOLO-(1,5-A)-PYRIMIDINE-7-YL )PHENYL)-ACETAMIDE

(30) Priorität: 14.02.2002 CH 250022002
(43) Veröffentlichungstag der Anmeldung: 17.11.2004
(73) Patentinhaber: Cilag AG, 8205 Schaffhausen (CH)
(72) Erfinder: HORNS, Stefan, CH-8200 Schaffhausen (CH)
(74) Vertreter: Braun, André jr.
(86) Internationale Anmeldenummer: PCT/CH2003/000026
(87) Internationale Veröffentlichungsnummer: WO 2003/068775

(56) Entgegenhaltungen:
- EP-A- 0 776 898

## Beschreibung

Die vorliegende Erfindung betrifft ein neues Verfahren zur Herstellung von N-Ethyl-N-[3-(3-cyanopyrazolo[1,5a]pyrimidin-7-yl)phenyl]acetamid der Formel III

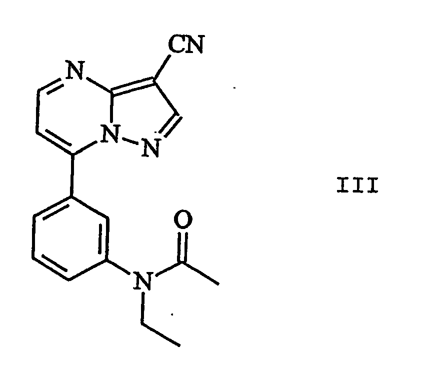

Dieses ist der Wirkstoff des unter dem Handelsnamen Sonata® zugelassenen Schlafmittels.

Das erfindungsgemässe Verfahren ist dadurch gekennzeichnet, dass man 3-(N-Acetyl-N-ethyl-amino)-β-oxo-phenylpropanal Natriumsalz der Formel I 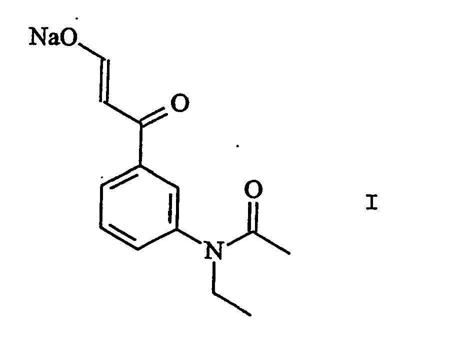 mit 3-Amino-4-cyanopyrazol der Formel II 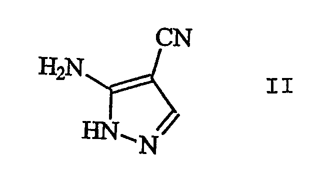 kondensiert.

Diese Kondensation erfolgt zweckmässigerweise in einem Gemisch von Eisessig, Ethanol, Isopropanol, Aceton oder Tetrahydrofuran mit konzentrierter Salzsäure und Wasser. Dabei besteht das Reaktionsmedium vorzugsweise aus etwa 150 bis 500, besonders bevorzugt etwa 300 bis 400, ganz besonders bevorzugt etwa 340 Gewichtsteilen Eisessig, Ethanol, Isopropanol, Aceton oder Tetrahydrofuran, etwa 30 bis 100, besonders bevorzugt etwa 45 bis 55, ganz besonders bevorzugt etwa 50 Gewichtsteilen konzentrierter (d.h. etwa 37%iger) Salzsäure und etwa 100 bis 400, besonders bevorzugt etwa 250 bis 300, ganz besonders bevorzugt etwa 270 Gewichtsteilen Wasser. Besonders bevorzugt als Reaktionsmedium ist ein Gemisch von Eisessig mit konzentrierter Salzsäure und Wasser, insbesondere ein Gemisch aus etwa 150 bis 500, besonders bevorzugt etwa 300 bis 400, ganz besonders bevorzugt etwa 340 Gewichtsteilen Eisessig, etwa 30 bis 100, besonders bevorzugt etwa 45 bis 55, ganz besonders bevorzugt etwa 50 Gewichtsteilen konzentrierter (d.h. etwa 37%iger) Salzsäure und etwa 100 bis 400, besonders bevorzugt etwa 250 bis 300, ganz besonders bevorzugt etwa 270 Gewichtsteilen Wasser. Beispielsweise kann man so vorgehen, dass man etwa 30 Gewichtsteile der Verbindung der Formel II in etwa 340 Gewichtsteilen Eisessig vorlegt, hierauf bei Raumtemperatur etwa 50 Gewichtsteile konzentrierte, d.h. etwa 37%ige Salzsäure zugibt und dann nach Kühlen auf etwa 5 bis 10°C, insbesondere auf etwa 7°C, langsam, zweckmässigerweise innerhalb etwa einer Stunde, eine Lösung von etwa 70 Gewichtsteilen des Natriumsalzes der Formel I in etwa 270 Gewichtsteilen Wasser zutropft, worauf man das Gemisch während geraumer Zeit, insbesondere während etwa einer Stunde unter Kühlung bei etwa der oben erwähnten Temperatur rührt. Anschliessend werden etwa 150 bis 800, zweckmässigerweise etwa 300 bis 500, vorzugsweise etwa 400 Gewichtsteile Wasser zugegeben, worauf das Produkt abfiltriert, mit Wasser gewaschen (zweckmässigerweise zweimal mit je etwa 150 Gewichtsteilen Wasser) und getrocknet werden kann, zweckmässigerweise im Vakuum bei etwa 50 bis 80, insbesondere bei etwa 60 bis 70°C. Die Verbindung der Formel III, d.h. das Zaleplon, kann so in guter Ausbeute und Reinheit in form eines weissen Feststottes erhalten werden.

Das als Ausgangsprodukt verwendete Natriumsalz der obigen Formel I kann erfindungsgemäss dadurch hergestellt werden, dass man 3-Acetylamino-acetophenon der Formel IV 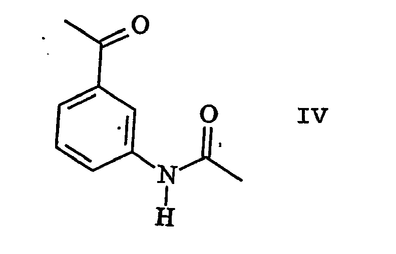 zunächst mit einem Alkalimetallhydroxid und dann mit einem Ethylierungsmittel behandelt und dann das erhaltene N-(3-Acetylphenyl)-N-ethyl-acetamid der Formel VII 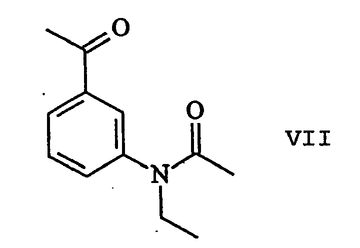 in Gegenwart eines Alkalimetallalkoholats mit einem Ameisensäurealkylester umsetzt.

Zweckmässigerweise verwendet man in der ersten dieser beiden Stufen als Alkalimetallhydroxid Kaliumhydroxid, und zwar vorzugsweise pulverisiertes festes Kaliumhydroxid, und als Ethylierungsmittel Ethylbromid. Vorzugsweise erfolgt diese Ethylierung in Tetrahydrofuran. Beispielsweise kann man so vorgehen, dass man etwa 100 Gewichtsteile 3-Acetylamino-acetophenon der Formel IV und etwa 45 bis 100, vorzugsweise etwa 65 Gewichtsteile pulverisiertes Kaliumhydroxid unter einer Inertgasatmosphäre, zweckmässigerweise unter Stickstoffatmosphäre, und unter Rühren in etwa 200 bis 500, vorzugsweise etwa 350 Gewichtsteilen wasserfreiem Tetrahydrofuran löst und dann bei etwa 35 bis 50°C, vorzugsweise etwa 40 bis 45°C etwa 100 bis 250, vorzugsweise etwa 155 Gewichtsteile gegebenenfalls mit Tetrahydrofuran verdünntes Ethylbromid zugibt. Nach einigen, zweckmässigerweise etwa 2 bis 15, vorzugsweise etwa 5 bis 6 Stunden kann das Gemisch auf Raumtemperatur gekühlt und mittels wässriger Säure, zweckmässigerweise mit verdünnter Salzsäure, neutralisiert werden; die organische Phase kann dann abgetrennt und zur Trockene eingeengt werden, worauf man den Rückstand beispielsweise dadurch trocknen kann, dass man die noch darin vorhandene Feuchtigkeit mittels Toluol o. dgl. azeotrop abdestilliert. Die Verbindung der Formel VII wird in hoher Ausbeute (gegen 95%) und befriedigender Reinheit (etwa 80 bis 92%) in Form eines Oels erhalten.

In der zweiten der beiden Stufen zur Herstellung des Natriumsalzes der Formel I verwendet man als Alkalimetallalkanolat zweckmäsigerweise Natriumethylat und als Ameisensäurealkylester zweckmässigerweise Ameisensäureethylester. Weiterhin erfolgt die Umsetzung zweckmässigerweise in Tetrahydrofuran. Beispielsweise kann man so vorgehen, dass man etwa 60 bis 65 Gewichtsteile des in der ersten Stufe erhaltenen Oels (Gehalt etwa 80 bis 92%) in etwa 120 bis 400, vorzugsweise etwa 200 bis 250 Gewichtsteilen wasserfreiem Tetrahydrofuran löst, worauf man unter Rühren bei Raumtemperatur etwa 25 bis 80, vorzugsweise etwa 35 bis 40 Gewichtsteile Ameisensäureethylester und etwa 120 bis 300, vorzugsweise etwa 160 bis 170 Gewichtsteile einer etwa 20 bis 25%igen Lösung von Natriumethanolat in Ethanol zugibt. Nach Rühren bei Raumtemperatur während einigen, zweckmässigerweise etwa 1 bis 24, vorzugsweise etwa 4 bis 6 Stunden kann man das Reaktionsgemisch gegebenenfalls mit weiterem Tetrahydrofuran versetzen und das ausgefallene Produkt, d.h. das Natriumsalz der Formel I, abfiltrieren, waschen (z.B. mit zweimal je etwa 45 Gewichtsteilen Tetrahydrofuran) und dann trocknen, zweckmässigerweise im Vakuum bei etwa 40 bis 50°C. Das Natriumsalz der Formel I kann so in guter Ausbeute in Form eines nicht hygroskopischen Pulvers erhalten werden.

In EP 0 208 864 B1 bzw. US 4,626,538 A wird beschrieben, dass man Zaleplon der obigen Formel III dadurch herstellen kann, dass man 3-Acetylamino-acetophenon der obigen Formel IV durch Umsetzung mit N,N-Dimethylformamid-dimethylacetal in N-[3-(3-Dimethylamino)-1-oxo-2-propenyl)phenyl]acetamid der Formel V 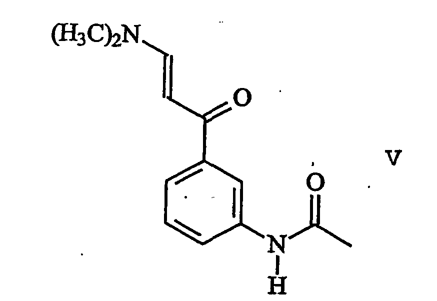 überführt, dieses in Gegenwart von Natriumhydrid mit einem Ethylhalogenid (z.B. Ethylbromid oder -iodid) in die entsprechende N-Ethylverbindung der Formel VI 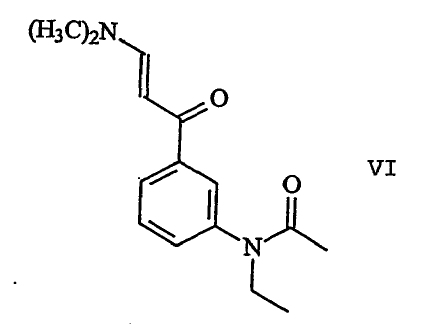 überführt und diese in Eisessig unter Rückfluss mit 3-Amino-4-cyanopyrazol der obigen Formel II kondensiert; gemäss einer Variante dieses Verfahrens kann man auch das N-[3-(3-Dimethylamino)-1-oxo-2-propenyl)phenyl]acetamid der obigen Formel V mit 3-Amino-4-cyanopyrazol der obigen Formel II kondensieren und dann das erhaltene Produkt ethylieren. Gemäss EP 0 776 898 A1 bzw. US 5,714,607 A erhält man Zaleplon als reineres und leichter isolierbares Produkt, wenn man die Kondensation der Verbindungen der obigen Formeln VI und II anstatt in Eisessig in einer Mischung von Wasser und Essigsäure durchführt, wobei der Wasseranteil zweckmässigerweise etwa 10 bis 85%, vorzugsweise etwa 11 bis 75%, insbesondere 60 bis 75% beträgt.

Wie die vorstehend diskutierten bekannten Verfahren geht auch das erfindungsgemässe Verfahren, wenn man die zum Natriumsalz der obigen Formel I führenden erfindungsgemässen Vorstufen mitberücksichtigt, letztlich von 3-Acetylamino-acetophenon aus. Das erfindungsgemässe Verfahren ist jedoch im Vergleich zu diesen bekannten Verfahren mit folgenden wesentlichen Vorteilen verbunden:
- Bei der Ethylierung (IV⇒VII im Vergleich zu V⇒VI) wird Kaliumhydroxid verwendet anstatt des erheblich teureren und mit hohen Sicherheitsrisiken behafteten Natriumhydrids.
- Anstatt N,N-Dimethylformamid-dimethylacetal (IV ⇒V) kann Ameisensäureethylester/Natriumethanolat (VII⇒I) verwendet werden, welche wesentlich preiswerter und in grösserem Massstab verfügbar sind.
- Das Natriumsalz der Formel I kann sehr leicht isoliert werden, denn es scheidet sich in hoher Ausbeute und Reinheit direkt aus dem Reaktionsgemisch ab und ist durch einfaches Abfiltrieren erhältlich.
- Während durch Kondensation der Verbindungen der Formeln VI und II in wässrigem Eisessig (vgl. EP 0 776 898 A1 bzw. US 5,714,607 A) erhaltenes Zaleplon der Formel III nach Aufarbeitung mit rund 1%, nämlich etwa 0.8 bis 1.3%, eines (sich im Reaktionsgemisch zu etwa 3 bis 6% bildenden) Isomeren kontaminiert ist, was eine aufwändige Reinigung erfordert, gelingt durch die erfindungsgemässe Kondensation der Verbindungen der Formeln I und II, insbesondere unter den weiter oben beschriebenen bevorzugten Reaktionsbedingungen, speziell unter den im nachfolgenden Beispiel 2 beschriebenen Bedingungen eine Minimierung dieses Nebenprodukts auf weniger als 0.1%.

Die nachfolgenden Beispiele sollen die vorliegende Erfindung näher illustrieren, ihren Umfang aber in keiner Weise einschränken.

### Beispiel 1

### a) Synthese des N-(3-Acetylphenyl)-N-ethyl-acetamids (VII)

100 g (0.564 mol) 3-Acetylamino-acetophenon, gefolgt von 63.2 g (1.13 mol) pulverisiertem Kaliumhydroxid werden unter N₂-Atmosphäre unter Rühren in wasserfreiem THF (350 g) gelöst und bei 40 - 45 °C mit einer Lösung von 154 g (1.41 mol) Ethylbromid in wasserfreiem THF (180 g) versetzt.
Nach 5 - 6 h wird auf Raumtemperatur gekühlt, und es werden 192.5 g (0.39 mol) 2 M HCl sowie 45 g Wasser zur Neutralisation zugegeben. Die organische Phase wird abgetrennt und zur Trockene eingeengt, und der Rückstand wird dann noch zweimal mit 175 g Toluol codestilliert.
Man erhält 133.4 g (Gehalt 82 %, Ausbeute 94 %) von Verbindung VII in Form eines Öls.

### b) Synthese des 3-(N-Acetyl-N-ethyl-amino)-β-oxo-phenylpropanal Na-Salz (I)

62.65 g (Gehalt 82 %, 0.250 mol) VII werden in wasserfreiem THF (220 g) gelöst, worauf unter Rühren bei Raumtemperatur 37.0 g (0.500 mol) Ameisensäureethylester, gefolgt von 162.1 g (0.500 mol) einer 21%igen Lösung von Natriumethanolat in Ethanol zugesetzt werden.
Nach 4- 6 h Rühren bei Raumtemperatur werden 220 g THF zugegeben, worauf das ausgefallene Produkt abfiltriert (Nutsche), mit zweimal 45 g THF nachgewaschen und im Vakuum bei 40 - 50 °C getrocknet wird.
Man erhält 61.16 g (85 %) von Verbindung I in Form eines Pulvers. Das Material ist nicht hygroskopisch.

### Beispiel 2

### Synthese des N-Ethyl-N-(3-(3-cyanopyrazolo-(1,5a)-pyrimidin-7-yl)phenyl)-acetamids (Zaleplon) (III)

Zu einer Lösung von 30.0 g (275 mmol) II in 340 g Eisessig werden bei Raumtemperatur 49.8 g (505 mmol) 37 %ige Salzsäure zugesetzt, und nach Kühlung auf 7 °C wird innerhalb von 1 h eine Lösung von 69.0 g (Gehalt 93 %, 251 mmol) I in 270 g Wasser zugetropft. Nach 1 h Rühren in der Kälte erfolgt Zugabe von 400 g Wasser, gefolgt von Abfiltrieren (Nutsche) und zweimaligem Waschen mit je 150 g Wasser. Das Produkt wird im Vakuum bei 60 - 70 °C getrocknet. Man erhält 69.6 g (88 %) von Verbindung III als weissen Feststoff.

## Patentansprüche

1. Verfahren zur Herstellung von N-Ethyl-N-[3-(3-cyanopyrazolo[1,5a]pyrimidin-7-yl)-phenyl]acetamid der Formel III 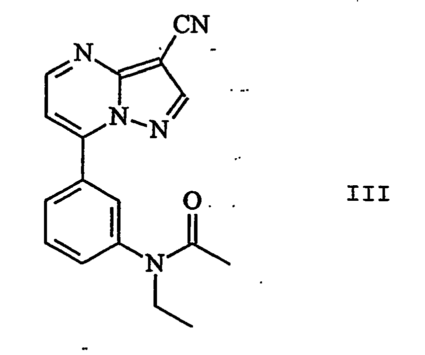 **dadurch gekennzeichnet, dass** man 3-(N-Acetyl-N-ethyl-amino)-β-oxophenylpropanal Natriumsalz der Formel I 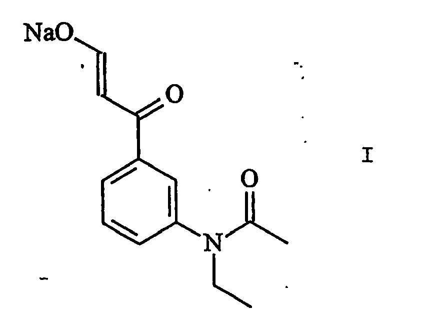 mit 3-Amino-4-cyanopyrazol der Formel II 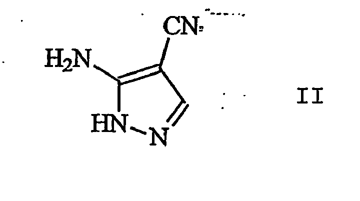 kondensiert.

2. Verfahren gemäss Anspruch 1, **dadurch gekennzeichnet, dass** die Kondensation in einem Gemisch von Eisessig, Ethanol, Isopropanol, Aceton oder Tetrahydrofuran mit konzentrierter Salzsäure und Wasser erfolgt.

3. Verfahren gemäss Anspruch 2, **dadurch gekennzeichnet, dass** das Reaktionsmedium ein Gemisch von etwa 150 bis 500 Gewichtsteilen Eisessig, Ethanol, Isopropanol, Aceton oder Tetrahydrofuran mit etwa 30 bis 100 Gewichtsteilen konzentrierter Salzsäure und etwa 100 bis 400 Gewichtsteilen Wasser ist.

4. Verfahren gemäss Anspruch 2, **dadurch gekennzeichnet, dass** das Reaktionsmedium ein Gemisch von Eisessig, konzentrierter Salzsäure und Wasser ist.

5. Verfahren gemäss Anspruch 4, **dadurch gekennzeichnet, dass** das Reaktionsmedium ein Gemisch von etwa 150 bis 500 Gewichtsteilen Eisessig, etwa 30 bis 100 Gewichtsteilen konzentrierter Salzsäure und etwa 100 bis 400 Gewichtsteilen Wasser besteht.

6. Verfahren gemäss einem der Ansprüche 1 bis 5, **dadurch gekennzeichnet, dass** das Ausgangsprodukt der Formel I **dadurch** hergestellt wird, dass man 3-Acetylamino-acetophenon der Formel IV 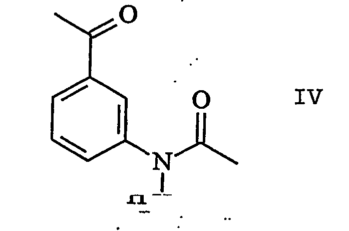 zunächst mit einem Alkalimetallhydroxid und dann mit einem Ethylierungsmittel behandelt und das erhaltene N-(3-Acetylphenyl)-N-ethyl-acetamid der Formel VII 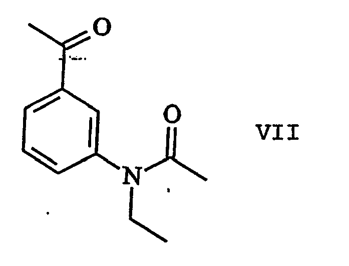 in Gegenwart eines Alkalimetallalkanolats mit einem Ameisensäurealkylester umsetzt.

7. Verfahren gemäss Anspruch 6, **dadurch gekennzeichnet, dass** man in der ersten Stufe als Alkalimetallhydroxid Kaliumhydroxid und als Ethylierungsmittel Ethylbromid verwendet.

8. Verfahren gemäss Anspruch 7, **dadurch gekennzeichnet, dass** die Ethylierung in Tetrahydrofuran erfolgt.

9. Verfahren gemäss einem der Ansprüche 6 bis 8, **dadurch gekennzeichnet, dass** man in der zweiten Stufe als Alkalimetallalkanolat Natriumethylat verwendet.

10. Verfahren gemäss Anspruch 9, **dadurch gekennzeichnet, dass** die Umsetzung in Tetrahydrofuran erfolgt.

## Claims

1. Process for the preparation of N-ethyl-N-[3-(3-cyanopyrazolo[1,5-a]-pyrimidin-7-yl)phenyl]acetamide of formula III: 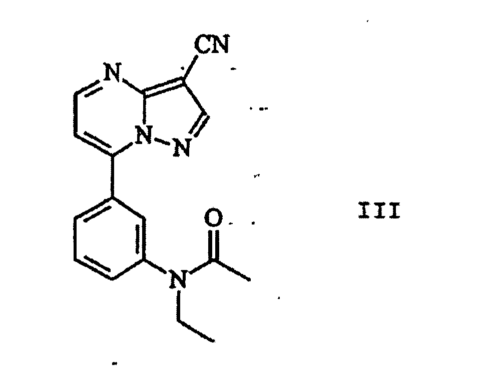 **characterized in that** the sodium salt of 3-(N-acetyl-N-ethylamino)-β-oxophenyl-propanal of formula I: 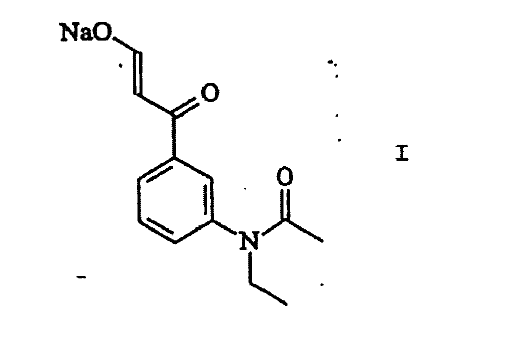 is condensed with 3-amino-4-cyanopyrazole of formula II:

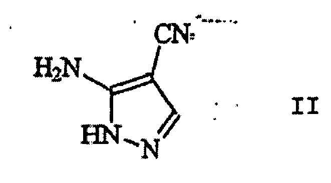

2. Process according to Claim 1, **characterized in that** the condensation reaction is carried out in a mixture of glacial acetic acid, ethanol, isopropanol, acetone or tetrahydrofuran with concentrated hydrochloric acid and water.

3. Process according to Claim 2, **characterized in that** the reaction medium is a mixture of about 150 to 500 parts by weight of glacial acetic acid, ethanol, isopropanol, acetone or tetrahydrofuran with about 30 to 100 parts by weight of concentrated hydrochloric acid and about 100 to 400 parts by weight of water.

4. Process according to Claim 2, **characterized in that** the reaction medium is a mixture of glacial acetic acid, concentrated hydrochloric acid and water.

5. Process according to Claim 4, **characterized in that** the reaction medium is a mixture of about 150 to 500 parts by weight of glacial acetic acid, about 30 to 100 parts by weight of concentrated hydrochloric acid and about 100 to 400 parts by weight of water.

6. Process according to one of Claims 1 to 5, **characterized in that** the starting material of formula I is prepared by treating 3-acetylaminoacetophenone of formula IV: 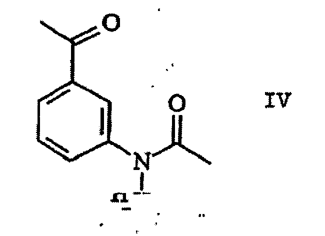 firstly with an alkali metal hydroxide and then with an ethylating agent, and reacting the resulting N-(3-acetylphenyl)-N-ethylacetamide of formula VII: 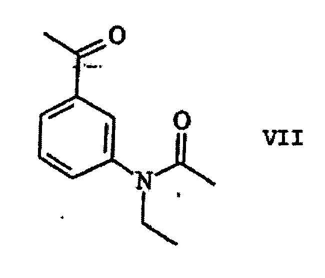 with an alkyl formate in the presence of an alkali metal alkanolate.

7. Process according to Claim 6, **characterized in that** the alkali metal hydroxide and ethylating agent used in the first stage are potassium hydroxide and ethyl bromide respectively.

8. Process according to Claim 7, **characterized in that** the ethylation is carried out in tetrahydrofuran.

9. Process according to one of Claims 6 to 8, **characterized in that** the alkali metal alkanolate used in the second stage is sodium ethylate.

10. Process according to Claim 9, **characterized in that** the reaction is carried out in tetrahydrofuran.

## Revendications

1. Procédé pour la préparation de N-éthyl-N-[3-(3-cyanopyrazolo[1,5a]pyrimidine-7-yl)-phényl]acétamide de la formule III 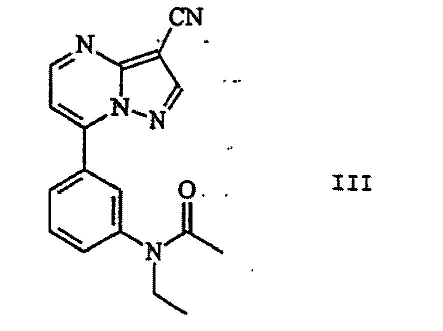 **caractérisé en ce que** l'on fait condenser du sel de sodium de 3-(N-acétyl-N-éthyl-amino)-β-oxo-phénylpropanal de la formule I 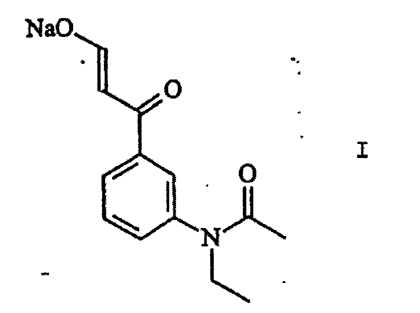 avec du 3-amino-4-cyanopyrazole de la formule Il.

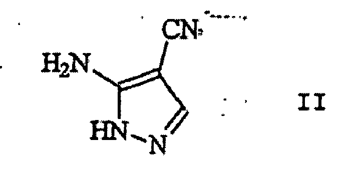

2. Procédé selon la revendication 1, **caractérisé en ce que** la condensation a lieu dans un mélange d'acide acétique glacial, éthanol, isopropanol, acétone ou tétrahydrofuranne avec de l'acide chlorhydrique concentré et de l'eau.

3. Procédé selon la revendication 2, **caractérisé en ce que** le médium de réaction est un mélange d'environ 150 à 500 parts en poids d'acide acétique glacial, éthanol, isopropanol, acétone ou tétrahydrofuranne avec environ 30 à 100 parts en poids d'acide chlorhydrique concentré et environ 100 à 400 parts en poids d'eau.

4. Procédé selon la revendication 2, **caractérisé en ce que** le médium de réaction est un mélange d'acide acétique glacial, d'acide chlorhydrique concentré et de l'eau.

5. Procédé selon la revendication 4, **caractérisé en ce que** le médium de réaction est un mélange d'environ 150 à 500 parts en poids d'acide acétique glacial, environ 30 à 100 parts en poids d'acide chlorhydrique concentré et environ 100 à 400 parts en poids d'eau.

6. Procédé selon l'une des revendications 1 à 5, **caractérisé en ce que** la matière de départ de la formule I est préparée en traitant du 3-acétylamino-acétophénon de la formule IV 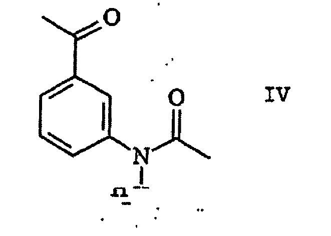 d'abord avec un hydroxyde de métal alcalin et ensuite avec un agent d'éthylation et en faisant réagir le N-(3-acétylphényl)-N-éthyl-acétamide de la formule VII obtenu 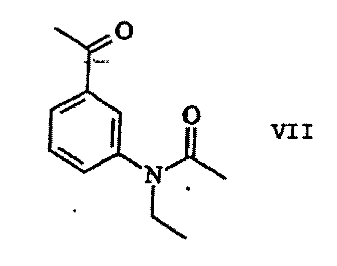 avec un ester alkylique d'acide formique en présence d'un alcanolate de métal alcalin.

7. Procédé selon la revendication 6, **caractérisé en ce que** dans la première étape de l'hydroxyde de potassium est utilisé en tant qu'hydroxyde de métal alcalin et du bromure d'éthyle est utilisé en tant qu'agent d'éthylation.

8. Procédé selon la revendication 7, **caractérisé en ce que** l'éthylation est réalisée dans du tétrahydrofuranne.

9. Procédé selon l'une des revendications 6 à 8, **caractérisé en ce que** dans la deuxième étape de l'éthylate de sodium est utilisé en tant qu'alcanolate de métal alcalin.

10. Procédé selon la revendication 9, **caractérisé en ce que** la réaction est réalisée dans du tétrahydrofuranne.
